# EUROPEAN PATENT APPLICATION

(11) **EP 4 640 661 A1**
(43) Date of publication of application: **29.10.2025**
(21) Application number: 24891718.9
(22) Date of filing: 08.11.2024
(51) Int. Cl.: C07C 29/80, C07C 29/82, C07C 29/86, C07C 29/04, C07C 31/10, C07C 31/12

(54) **METHOD FOR PURIFYING ISOPROPYL ALCOHOL**

(30) Priority: 15.11.2023 KR 20230158482; 25.10.2024 KR 20240147844
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR); LEE, Moon Yong, Daejeon 34122 (KR)
(74) Representative: Goddar, Heinz J.
(86) International application number: PCT/KR2024/017591
(87) International publication number: WO 2025/105772

(57) **Abstract**

The present invention provides a method for purifying isopropyl alcohol, the method including: supplying a feed including isopropyl alcohol, water, a first light by-product, a second light by-product, and a heavy by-product to a first column and separating the first light by-product; and supplying a lower discharge stream of the first column to a second column and separating the lower discharge stream of the first column into each of an upper discharge stream of the second column including the second light by-product, a first side discharge stream of the second column including a mixture of the isopropyl alcohol and water, a second side discharge stream of the second column including the heavy by-product, and a lower discharge stream of the second column including the water.

## Description

### [Technical Field]

### CROSS-REFERENCE TO RELATED APPLICATION

The present application claims the benefit of priorities to Korean Patent Application No. 10-2023-0158482, filed on November 15, 2023, and Korean Patent Application No. 10-2024-0147844, filed on October 25, 2024, the entire contents of which are incorporated herein by reference.

### Technical Field

The present invention relates to a method for purifying isopropyl alcohol, and in particular, to a method for purifying isopropyl alcohol from a reaction product of an isopropyl alcohol preparation process, which may reduce energy consumption and process costs.

### [Background Art]

Isopropyl alcohol (IPA) is recognized as an excellent solvent in a variety of industries and applications because it has an ability to dissolve a wide range of substances, rapid vaporization, and relatively low toxicity. Isopropyl alcohol is an essential substance in a variety of manufacturing industries, health care, and direct consumer applications.

In an isopropyl alcohol preparation process, for example, propylene and water are used as raw material components. In this case, the propylene and water react to produce isopropyl alcohol. The reaction product of the isopropyl alcohol preparation process includes various types of impurities and by-products such as diisopropyl ether (DIPE), acetone, n-propyl alcohol (NPA), and hexanol, in addition to isopropyl alcohol, an unreacted propylene monomer, and unreacted water.

In order to obtain isopropyl alcohol from the reaction product, a purification process of isopropyl alcohol is required. Therefore, in order to obtain high-purity isopropyl alcohol, the purification process of isopropyl alcohol needs to be highly efficient, and at the same time, an improved design is required from an economic perspective that may reduce not only energy consumption but also operating costs and facility costs.

### [Disclosure]

### [Technical Problem]

In order to solve the problems mentioned in the background art, an object of the present invention is to provide a method for purifying isopropyl alcohol that may obtain high-purity isopropyl alcohol, and at the same time, may reduce energy consumption and improve operating costs and facility costs.

However, the problems to be solved by the present application are not limited to the object described above, and other problems not described will be clearly understood by those skilled in the art from the following description.

### [Technical Solution]

In one general aspect, a method for purifying isopropyl alcohol includes: supplying a feed including isopropyl alcohol, water, a first light by-product, a second light by-product, and a heavy by-product to a first column; separating the first light by-product through an upper discharge stream of the first column; supplying a lower discharge stream of the first column including the isopropyl alcohol, water, second light by-product, and heavy by-product to a second column, and separating the lower discharge stream of the first column into each of an upper discharge stream of the second column including the second light by-product, a first side discharge stream of the second column including a mixture of the isopropyl alcohol and water, a second side discharge stream of the second column including the heavy by-product, and a lower discharge stream of the second column including the water; and introducing the mixture of the isopropyl alcohol and water into an isopropyl alcohol recovery process and separating the isopropyl alcohol and water.

### [Advantageous Effects]

According to the method for purifying isopropyl alcohol of the present invention, a feed including isopropyl alcohol, water, and various by-products as a reaction product of propylene and water is effectively purified, such that a mixture (azeotrope) of isopropyl alcohol and water in which a content of by-products is minimized may be obtained. The mixture of isopropyl alcohol and water in which a content of by-products is minimized is subjected to a subsequent isopropyl alcohol recovery process, such that isopropyl alcohol with a high purity may be finally obtained.

In addition, a process of obtaining a mixture of isopropyl alcohol and water is performed in one distillation column, which has been performed by at least two distillation columns in the related art, such that energy efficiency may be realized in the overall preparation process of isopropyl alcohol. That is, according to the present invention, the reboiler duty required for the operation of at least two distillation columns may be saved (energy saving) in the related art, and the facility costs and operating costs of the device may be saved by reducing the number of distillation columns.

### [Description of Drawings]

FIG. 1 is a process flow diagram of a method for purifying isopropyl alcohol according to an embodiment of the present invention.
FIG. 2 is a process flow diagram of a method for purifying isopropyl alcohol according to a comparative example.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

With regard to the description of the drawings, similar reference numerals may be used to refer to similar or related components.

A singular form of a noun corresponding to an item may include one or more of the items, unless the relevant context clearly indicates otherwise.

In the present disclosure, each of such phrases as "A or B", "at least one of A and B", "at least one of A or B", "A, B, or C", "at least one of A, B, and C", and "at least one of A, B, or C" may include any one of or all possible combinations of the items enumerated together in one of the corresponding phrases.

The term "and/or" includes a combination of a plurality of related components or any one of the plurality of related components.

The terms such as "1st" and "2nd" or "first" and "second" may be used to simply distinguish a corresponding component from another component, and do not limit the corresponding components in other aspects (for example, importance or order).

In addition, the terms such as "front surface", "rear surface", "upper surface", "lower surface", "side surface", "left side", "right side", "upper portion", and "lower portion" used in the present application are defined based on the drawings, and the shape and position of each component are not limited by the terms.

The term "include" or "have" specifies the presence of features, numerals, steps, operations, components, parts described in the present disclosure, or a combination thereof, but does not preclude the presence or addition of one or more other features, numerals, steps, operations, components, parts, or a combination thereof.

When a component is said to be "connected", "coupled", "supported", or "contacted" with another component, this includes not only when components are directly connected, coupled, supported, or contacted, but also when components are indirectly connected, coupled, supported, or contacted through a third component.

When a component is said to be "on" another component, this includes not only when the component is in contact with another component, but also when there is another component between two components.

The term "stream" used in the present application may refer to a flow of a fluid in a process, and may also refer to a fluid flowing through a pipe itself. Specifically, the stream may refer to both a fluid flowing through a pipe connecting respective devices to each other itself and a flow of a fluid. In addition, the fluid may include any one or more components of gas, liquid, and solid.

The term "upper portion" used in the present application refers to a point at a height of 0% to 10% from the uppermost portion of a device downwards, unless otherwise specified, and specifically, may refer to the uppermost portion (the top). In addition, the term "lower portion" refers to a point at a height of 90% to 100% from the uppermost portion of the device downwards, and specifically, may refer to the lowest portion (the bottom).

In addition, the term "pressure" as referred to in the present application refers to a gauge pressure measured under atmospheric pressure conditions.

Meanwhile, unless otherwise specified herein, an operating pressure of a column refers to a pressure at an upper portion of the column, and an operating temperature of the column refers to a temperature at a lower portion of the column.

An embodiment of the present invention relates to a method for purifying isopropyl alcohol (IPA). Hereinafter, the method for purifying isopropyl alcohol of the present invention will be described in detail with reference to the drawings.

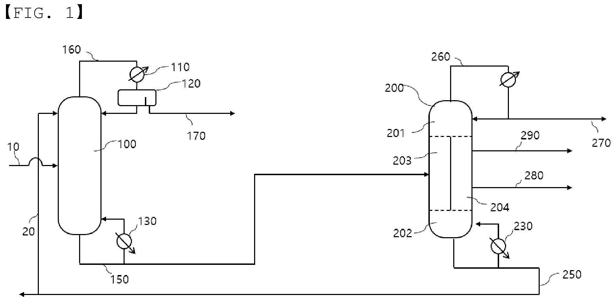

FIG. 1 is a process flow diagram of a method for purifying isopropyl alcohol according to an embodiment of the present invention.

A method for purifying isopropyl alcohol according to the present invention includes supplying a feed 10 including isopropyl alcohol, water, a first light by-product, a second light by-product, and a heavy by-product to a first column 100.

First, the feed 10 may be derived from a reaction product including isopropyl alcohol through a gas phase reaction of a propylene monomer and water performed in a reaction section. Specifically, the feed 10 may be a residue obtained by separating gas components including unreacted propylene included in the reaction product.

There are various methods for recovering (unreacted) propylene from the reaction product to prepare the feed 10 of the present invention. Hereinafter, among the various methods for recovering propylene from the reaction product, a method for recovering propylene with a high purity and recycling the recovered propylene to a reaction section in which the gas phase reaction is performed is presented as an example.

Since propylene is recycled to the reaction section and used again as a raw material for the gas phase reaction, unreacted propylene after the reaction needs to be recovered with a high purity in order to prepare high-purity isopropyl alcohol. Specifically, it is preferable that propylene (C₃H₆) to be supplied to the reaction section as a raw material has a high purity as described above. In a case where impurities such as other unsaturated hydrocarbons, for example, ethylene, butene, pentene, and the like, ethane, propane, and carbon dioxide are included in the raw material propylene, by-products (for example, ethanol) having a boiling point similar to that of isopropyl alcohol may be produced during the reaction between propylene and water. Therefore, it is preferable that the propylene supplied as a reactant to the reaction section includes propylene in an amount of 97 wt% or more, for example, 97 to 99.8 wt%, with respect to the total weight thereof, and a content of impurities is less than 3 wt%.

Meanwhile, only a portion of the propylene supplied to the reaction section is used for the reaction. Accordingly, the reaction product may include unreacted propylene and unreacted water in addition to isopropyl alcohol produced by the reaction of the propylene monomer and water. For example, the reaction product may include 65 to 85 wt% of a propylene monomer, 4 to 8 wt% of isopropyl alcohol, and 5 to 30 wt% of water. In addition, the reaction product may include at least two or more types of light by-products and heavy by-products as by-products. Specifically, the light by-product may include diisopropyl ether (DIPE) and acetone, and the heavy by-product may include n-propyl alcohol (NPA) and hexanol. Therefore, there is a need for performing a process of separating unreacted raw materials from the reaction product and purifying isopropyl alcohol from various by-products.

According to an embodiment of the present invention, recovery of propylene in the reaction product may be performed by a gas purifying section provided with two or more of an absorption column, a flash drum, and a gas purification column.

Specifically, propylene is separated by supplying the reaction product to a lower portion of the absorption column and introducing water into an upper portion of the absorption column. In this case, the water may be water supplied from a lower discharge stream of a second column described below. In the absorption column, gas phase isopropyl alcohol included in the reaction product may be absorbed into the water and obtained through a liquid phase lower stream, and a gas phase stream including propylene may be separated through the upper portion of the absorption column. The propylene included in the gas phase stream may be recycled to the reaction section.

Meanwhile, the liquid phase lower stream of the absorption column may include a small amount of low-boiling-point gas components including propylene that has not been separated in the absorption column, in addition to isopropyl alcohol and water. Therefore, the liquid phase stream including isopropyl alcohol separated from the absorption column may be supplied to the flash drum to additionally recover propylene. For example, the liquid phase stream may be supplied to one or more flash drums operated under reduced pressure conditions to recover low-boiling-point gas components including propylene included in the liquid phase stream as gas, and then supplied to the gas purification column to additionally recover propylene that may remain in the liquid phase stream including isopropyl alcohol.

Through such a process, a gas phase upper stream including propylene and a liquid phase lower stream including isopropyl alcohol and water may be separated from the absorption column, the flash drum, and the gas purification column.

Meanwhile, the liquid phase lower stream separated from the absorption column, the flash drum, and the gas purification column may include a first light by-product including diisopropyl ether (DIPE), a second light by-product including acetone, and a heavy by-product including n-propyl alcohol (NPA) and hexanol, in addition to isopropyl alcohol and water. As described above, the liquid phase lower stream separated from one or more of the absorption column, the flash drum, and the gas purification column for recovery of propylene may be the feed 10 of the present invention supplied to the first column 100.

The feed 10 may be introduced into a point at a height of 30 to 50% downwards from the top of a first column 100.

According to an embodiment of the present invention, the first light by-product included in the feed 10 may be first separated and removed by a layer separator 120 connected to the first column 100 and an upper portion of the first column 100.

Specifically, an upper discharge stream 160 of the first column including isopropyl alcohol, water, the first light by-product, and optionally the second light by-product and a lower discharge stream 150 of the second column including isopropyl alcohol, water, the second light by-product, and the heavy by-product may be discharged through the upper portion and the lower portion of the first column 100, respectively, by the distillation in the first column 100.

The upper discharge stream 160 of the first column may be supplied to a condenser 110 and cooled and liquefied after being discharged from the first column 100. The liquefied upper discharge stream of the first column may be supplied to the layer separator 120 and subjected to liquid-liquid separation. By the liquid-liquid separation, a water phase stream including isopropyl alcohol, water, and optionally the second light by-product may be refluxed to the first column, and an oil phase stream 170 including the first light by-product may be discharged outside the system. The amount of the first light by-product discharged outside the system may be 97 wt% or more, 99 wt% or more, and specifically, 100 wt%, when the content of the first light by-product included in the feed 10 is 100 wt%.

In order for the first light by-product to be easily separated by distillation in the first column 100 and liquid-liquid separation in the layer separator 120 provided above the first column, at least the first light by-product should be an oily component that does not dissolve in water. That is, the first column 100 is operated under operating conditions in which a heavy by-product is not vaporized, and the first light by-product is substantially separated from water and isopropyl alcohol dissolved in water in the layer separator 120, such that the first light by-product may be efficiently separated.

According to an embodiment of the present invention, the first light by-product may be diisopropyl ether (DIPE) which is insoluble in water, and the second light by-product may be acetone which is soluble in water. Since a boiling point of acetone is lower than that of diisopropyl ether, the upper discharge stream 160 of the first column may include water, isopropyl alcohol, acetone, and diisopropyl ether (DIPE). The isopropyl alcohol included in the upper discharge stream 160 of the first column is separated from the first light by-product (oil phase) by liquid-liquid separation performed in the layer separator 120, and water, isopropyl alcohol, and acetone included in the water phase are refluxed back to the first column 100. Therefore, the loss of isopropyl alcohol at the upper portion of the first column 100 may be minimized by the layer separator 120 provided above the first column 100.

Meanwhile, almost all of the first light by-product included in the feed 10 may be discharged outside the system. To this end, the operating conditions of the first column 100 should be controlled so that almost all of the first light by-product included in the feed 10 may be included in the upper discharge stream 160 of the first column 100.

Specifically, an operating temperature of the first column 100 may be 75°C or higher or 80°C or higher and 95°C or lower or 90°C or lower. The operating temperature may refer to a temperature at the lower portion of the first column 100. Meanwhile, an operating pressure of the first column 100 may be 1 kg/cm²·g or less or 0.5 g/cm²·g or less. The operating pressure may refer to a pressure at the upper portion of the first column 100. When the first column 100 is operated at the operating temperature and the operating pressure as described above, the first light by-product may be separated as much as possible through the upper discharge stream 160 of the first column, and therefore, the first light by-product may be prevented from flowing out through the lower discharge stream 150 of the first column and remaining as an impurity in isopropyl alcohol prepared as a result.

Meanwhile, in order to effectively recover the isopropyl alcohol included in the upper discharge stream 160 of the first column and reflux the recovered isopropyl alcohol back to the first column by the liquid-liquid separation performed in the layer separator 120, water should be supplied to the upper portion of the first column. Since a significant amount of water is discharged through the lower discharge stream 150 of the first column, it is preferable to supply water to the first column 100 in order to sufficiently dissolve isopropyl alcohol in the layer separator 120 to form a water phase. The water supplied to the upper portion of the first column is supplied to the first column separately from the feed 10. The water supplied to the upper portion of the first column may be water included in a stream 20 that is branched and recycled from a portion of a lower discharge stream 250 of the second column including water as described below.

As described above, since the water recycled from the second column 200 to the first column 100 includes almost no heavy by-product due to the excellent separation ability in the second column, particularly, the ability to effectively separate the heavy by-product, it is possible to recycle the lower discharge stream 250 of the second column back to the first column.

It is preferable that the water supplied to the first column 100 is supplied to the upper portion of the first column 100. This is because the water supplied to the upper portion may dissolve isopropyl alcohol, which is highly soluble in water, as it moves from the upper portion to the lower portion, and may be discharged through the lower discharge stream 150 of the first column. If the second light by-product is also soluble in water, the isopropyl alcohol and the second light by-product may be dissolved in water and discharged through the lower discharge stream 150 of the first column.

Meanwhile, according to an embodiment of the present invention, a mass flow rate of the branched stream 20 of the lower discharge stream 250 of the second column recycled to the upper portion of the first column needs to be controlled from the viewpoint of the loss of isopropyl alcohol in the layer separator 120 and the energy consumption in the first column 100. Specifically, the mass flow rate of the branched stream 20 of the lower discharge stream 250 of the second column may be 0.4 to 1.2, 0.4 to 1.0, or 0.5 to 0.8, with respect to a mass flow rate of the feed 10 supplied to the first column 100. When a flow rate of the water supplied to the upper portion of the first column is larger than 1.2, the energy consumption required in the first column excessively increases. On the other hand, when the flow rate of the water supplied to the upper portion of the first column is less than 0.4, it is difficult to supply sufficient water to the layer separator 120, and thus, the loss of isopropyl alcohol in an oil phase may occur or may increase excessively.

Meanwhile, in the layer separator 120 provided above the first column 100, isopropyl alcohol should be included in the water phase and refluxed together with water to the first column 100. In a case in which the isopropyl alcohol is included in the oil phase, the loss of isopropyl alcohol occurs in the layer separator 120, and in order to prevent the loss of isopropyl alcohol, a sufficient amount of water should be secured in the layer separator 120. The amount of water in the layer separator 120 is affected by the amount of water introduced into the first column 100.

According to an embodiment of the present invention, the water introduced into the first column 100 may be the water included in the feed 10 and the water included in the stream 20 recycled after being branched from a portion of the lower discharge stream 250 of the second column. In order to minimize the loss of isopropyl alcohol in the layer separator 120, it is preferable that the sum of a mass flow rate of the water included in the feed 10 and a mass flow rate of the water included in the stream 20 recycled after being branched from a portion of the lower discharge stream 250 of the second column is maintained to be 12 to 15 times (the ratio of the mass flow rates of isopropyl alcohol and water) the mass flow rate of the isopropyl alcohol included in the feed 10. In this case, a sufficient amount of water may be supplied to the layer separator 120, the loss of isopropyl alcohol in an oil phase in the layer separator 120 may be prevented, and at the same time, the energy consumption required for the operation of the first column 100 may be optimized.

Specifically, the branched stream 20 branched from a portion of the lower discharge stream of the second column including water may be a branched stream branched from a portion of a stream that is refluxed to a reboiler 230 among the streams immediately after being discharged to the lower portion of the second column.

That is, when a ratio of the mass flow rates of the water and isopropyl alcohol supplied to the first column is less than 12, it is difficult to secure a sufficient amount of water in the layer separator 120, such that the loss of isopropyl alcohol in an oil phase occurs, and it is difficult to achieve a desired recovery rate of isopropyl alcohol. Furthermore, in this case, a portion of the first light by-product that should be removed while included in an oil phase in the layer separator 120 is included in a water phase and introduced into the second column 200, and in a case in which the first light by-product is introduced into the second column 200, the first light by-product is also included in the first side discharge stream of the second column including a mixture of isopropyl alcohol and water, and the purity of isopropyl alcohol recovered in the second column is consequently reduced.

In addition, a ratio of the mass flow rates of water and isopropyl alcohol supplied to the first column is larger than 15, the loss of isopropyl alcohol in the layer separator 120 may be prevented, but the amount of water recycling through the first column 100 and the second column 200 is excessively large, which may increase the energy consumption in the two columns.

Furthermore, from the viewpoint of minimizing the loss of isopropyl alcohol in the layer separator 120 and reducing the energy consumption in the first and second columns, the mass flow rate of the water included in the stream 20 recycled after being branched from a portion of the lower discharge stream 250 of the second column may be 58% to 90% based on the mass flow rate of the water included in the feed 10.

Meanwhile, a reboiler 130 supplying heat energy required for the operation of the first column is provided below the first column 100. A reflux stream of the lower discharge stream 150 of the first column may be introduced again into the reboiler 130, heat-exchanged with a high-temperature heat source, and then introduced again into the lower portion of the first column 100. Heat energy required for the operation of the first column 100 may be supplied to the first column 100 through the reboiler 130.

According to an exemplary embodiment of the present invention, the lower discharge stream 150 of the first column is introduced into the second column 200, and the second light by-product, the mixture of isopropyl alcohol and water, the heavy by-product, and water may be separated according to boiling point.

Specifically, a step of supplying the lower discharge stream 150 of the first column including the isopropyl alcohol, water, second light by-product, and heavy by-product and separating the lower discharge stream of the first column into each of an upper discharge stream 260 of the second column including the second light by-product, a first side discharge stream 290 of the second column including the mixture of isopropyl alcohol and water, a second side discharge stream 280 of the second column including the heavy by-product, and a lower discharge stream 250 of the second column including the water may be performed.

The second light by-product is a by-product having the relatively lowest boiling point compared to other separated components, and the second light by-product may be a compound having a boiling point of 50 to 70°C, and specifically, may be acetone. The acetone may be a by-product produced during a gas phase reaction for preparing isopropyl alcohol, and may be a by-product produced by oxidation of isopropyl alcohol in a subsequent process after the gas phase reaction.

The upper discharge stream 260 of the second column may include 60 wt% or more, 70 wt% or more, or 90 wt% or more, and 100 wt% or less of the second light by-product, and may include the residual mixture of isopropyl alcohol and water. After the upper discharge stream 260 of the second column is discharged from the second column, a portion of the upper discharge stream of the second column may be refluxed back to the second column after passing through the condenser, and the remainder may be discharged outside the system 270.

Meanwhile, the mixture of isopropyl alcohol and water may be an azeotrope of isopropyl alcohol and water. That is, water having a boiling point of about 100°C and isopropyl alcohol having a boiling point of about 82.3°C may form an azeotrope at an azeotropic temperature of about 81°C. A boiling point of the azeotrope of isopropyl alcohol and water is higher than a boiling point of the second light by-product and is lower than a boiling point of the heavy by-product.

Therefore, a portion of the water introduced into the second column forms an azeotrope with isopropyl alcohol and is discharged through the first side discharge stream 290 of the second column, and the remaining water is discharged through the lower discharge stream 250 of the second column.

The reboiler 230 supplying heat energy required for the operation of the second column is provided below the second column 200. A reflux stream of the lower discharge stream of the second column may be introduced into the reboiler 230 and heat-exchanged with a high-temperature heat source, and then introduced again into the lower portion of the second column 200. Heat energy required for the operation of the second column 200 may be supplied to the second column 200 through the reboiler 230.

Meanwhile, the branched stream 20 branched from a portion of the lower discharge stream 250 of the second column including water may be recycled to the upper portion of the first column. The water recycled from the second column 200 may be used to supplement a sufficient amount of water so that the phase separation between the water phase and the oil phase may be smoothly performed in the layer separator 120 provided above the first column 100.

Meanwhile, the heavy by-product may include n-propyl alcohol (NPA) and hexanol, and these heavy by-products may be discharged through the second side discharge stream 280 of the second column.

As described above, according to an embodiment of the present invention, the second column 200 includes a dividing wall spaced apart from the bottom and provided in a longitudinal direction of the column, and the second column may be a distillation column partitioned into a top region 201, a bottom region 202, a supply region 203, and a discharge region 204 by the dividing wall.

Referring to FIG. 1, the inside of the second column 200 is divided by the dividing wall and the imaginary dotted line. Specifically, the top region 201 is a region located above an upper end of the dividing wall and is a region from which the upper discharge stream 260 of the second column is discharged, and the bottom region 202 is a region located below a lower end of the dividing wall and is a region from which the lower discharge stream 250 of the second column is discharged. Meanwhile, the lower discharge stream 150 of the first column may be supplied to the supply region 203.

The first side discharge stream 290 of the second column and the second side discharge stream 280 of the second column may be discharged from the discharge region 204 among the regions partitioned by the dividing wall. Specifically, the first side discharge stream 290 may be discharged from the discharge region at a higher position than the second side discharge stream 280.

That is, according to an embodiment of the present invention, the composition (the lower discharge stream of the first column) including at least four components such as isopropyl alcohol, water, a second light by-product, and a heavy by-product is separated and discharged through the upper portion, the first side, the second side, and the lower portion of one column (the second column) provided with a dividing wall, such that the number of distillation columns previously required for separation of these components may be reduced.

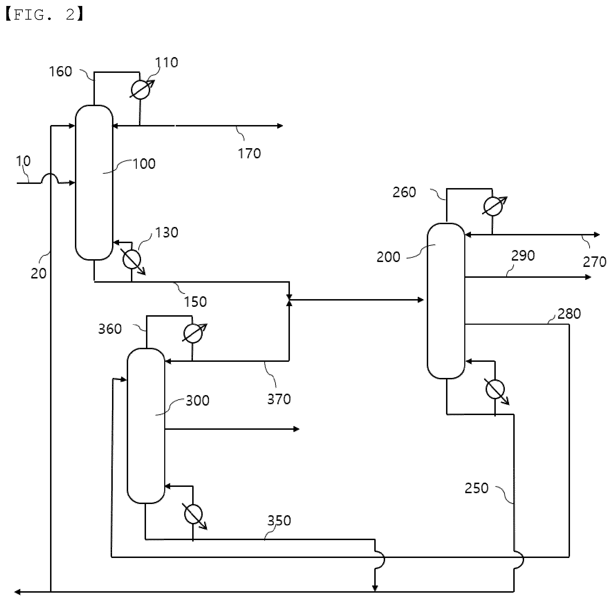

Specifically, referring to FIG. 2 in which a distillation column without a dividing wall is used as the second column 200, although it is not impossible to discharge the separated material through an upper portion, a lower portion, and first and second sides of the second column 200, in particular, the second side discharge stream 280 through which a heavy by-product is separated includes a large amount of isopropyl alcohol and water; thus, additional purification of the second side discharge stream 280 is required to increase a yield of isopropyl alcohol. That is, it is required to perform a process of introducing the second side discharge stream 280 into a third column 300, additionally recovering a stream 370 including isopropyl alcohol to the upper portion, and supplying the recovered stream again to the second column 200. That is, referring to FIG. 2, according to the present invention, the same roles performed by the conventional second column 200 and third column 300 may be performed by one column including a dividing wall, such that the number of columns may be reduced, and the energy consumption (for example, steam consumption) required for the operation of the column may be reduced.

Meanwhile, according to the process of purifying isopropyl alcohol of the present invention, a yield of the isopropyl alcohol may refer to a ratio of a mass flow rate of the isopropyl alcohol included in the first side discharge stream (the azeotrope of isopropyl alcohol and water is discharged) of the second column to a mass flow rate (for example, ton/hr) of the isopropyl alcohol included in the feed 10, and the yield of the isopropyl alcohol may be 95 wt% or more or 97 wt% or more and 99.9 wt% or less or 99.5 wt% or less. Therefore, in the case of the present invention, an excellent yield of isopropyl alcohol may be achieved using only the second column without additional purification of the second side discharge stream (including a heavy by-product).

Therefore, according to the present invention, since a separate column for additional purification of the second side discharge stream including a heavy by-product is not required, the number of columns for purification of isopropyl alcohol may be reduced compared to the related art illustrated in FIG. 2. That is, as the number of columns performing the same role is reduced, the energy consumption required in the reboiler in each column may be reduced, and at the same time, as the second column 200 including a separation wall is used, the preliminary separation occurs in the supply region 203 and the final purification is performed in the discharge region 204, such that energy savings may be achieved beyond simply operating two combined columns.

Meanwhile, the upper end of the dividing wall may be located at a height of 3% to 30% downwards from the top of the second column, and the lower end of the dividing wall may be located at a height of 70% to 95% downwards from the top of the second column.

Furthermore, the first side discharge stream 290 may be discharged at a height of 5 to 33% downwards from the top of the second column, and the second side discharge stream 280 may be discharged at a height of 40 to 80% downwards from the top of the second column.

Through the location of the dividing wall and the discharge points of the first and second side discharge streams, the energy consumption may be reduced compared to when using two conventional columns, and at the same time, four streams discharged from the second column may be obtained with a desired purity.

An operating temperature and an operating pressure of each of the top region 201 and the bottom region 202 of the second column 200 also need to be controlled from the viewpoint of the purity of the separated components such as isopropyl alcohol and the energy required to separate these components.

Specifically, the operating temperature of the top region 201 of the second column 200 may be 90°C or lower, 85°C or lower, or 80°C or lower, and the operating pressure of the top region 201 may be 2 kg/cm²·g or less, 1 kg/cm²·g or less, or 0.05 kg/cm²·g or less.

Meanwhile, the operating temperature of the bottom region 202 of the second column 200 may be 85°C or higher or 88°C or higher and 105°C or lower or 103°C or lower. Meanwhile, the operating pressure of the bottom region 202 may be 1.0 kg/cm²·g or less or 0.5 kg/cm²·g or less.

The method for purifying isopropyl alcohol according to an embodiment of the present invention may include introducing the first side discharge stream 290 of the second column including the mixture of the isopropyl alcohol and water into an isopropyl alcohol recovery process and separating the isopropyl alcohol and water.

As described above, the first side discharge stream 290 of the second column may include a mixture of isopropyl alcohol and water, and specifically, an azeotrope of isopropyl alcohol and water. More specifically, the first side discharge stream 290 of the second column may include 80 to 90 wt% of isopropyl alcohol and 10 to 20 wt% of water.

Thereafter, the first side discharge stream 290 of the second column may be supplied to an IPA recovery column and may be separated into a stream including water and an azeotropic agent and a stream including isopropyl alcohol in the presence of an azeotropic agent (for example, cyclohexane, benzene, or the like). That is, isopropyl alcohol purified to a high purity may be obtained by removing the azeotrope of isopropyl alcohol and water by the azeotropic agent in the IPA recovery column.

The stream including the water and the azeotropic agent separated from the IPA recovery column may be separated into each of water and the azeotropic agent in a separate distillation column (for example, a solvent recovery column), and the separated azeotropic agent may be supplied back to the IPA recovery column and reused for separating isopropyl alcohol and water.

### [Mode for Invention]

Hereinafter, the present invention will be described in more detail with reference to examples. However, the following examples are provided for illustrating the present invention. It is apparent to those skilled in the art that various modifications and alterations may be made without departing from the scope and spirit of the present invention, and the scope of the present invention is not limited thereto.

In the following examples and comparative examples, the method according to the present invention was simulated using the commercial process simulation program Aspen Plus V12.1.

### Example 1

A process of purifying isopropyl alcohol was performed according to a process diagram of FIG. 1.

Specifically, water and propylene were supplied to a reaction section and subjected to a gas phase reaction to produce a reaction product including isopropyl alcohol, water, and propylene. The propylene was separated and recovered from the reaction product, and a feed 10 was prepared from the remaining residue. Each of 10.395 wt% of isopropyl alcohol, 88.9 wt% of water, 0.5 wt% of diisopropyl ether (DIPE) as a first light by-product, 0.005 wt% of acetone as a second light by-product, and 0.2 wt% of n-propyl alcohol (NPA) and hexanol as heavy by-products was included in the feed 10.

The feed 10 was supplied to a first column, and water 20 recycled in a second column was supplied to an upper portion of the first column. At this time, a portion of a lower discharge stream of the second column 200 was branched and introduced into the upper portion of the first column 100, and a mass flow rate of the branched stream 20 branched from a portion of a lower discharge stream of the second column 200 was 0.6 with respect to a mass flow rate of the feed 10 supplied to the first column 100.

Meanwhile, the heat required for the operation of the first column was supplied through a reboiler 130.

A stream 160 including isopropyl alcohol, water, diisopropyl ether (DIPE), and acetone was discharged through the upper portion of the first column 10 and supplied to a condenser 110 and a layer separator 120, a water phase including isopropyl alcohol, water, and acetone was refluxed back to the first column 10, and an oil phase including diisopropyl ether was discharged outside the system.

A lower discharge stream of the first column was introduced into a supply region 203 of the second column including a dividing wall, and separated, by distillation, into each of four discharge streams: an upper discharge stream 260 of the second column including acetone, a first side discharge stream 290 of the second column including an azeotrope of isopropyl alcohol and water, a second side discharge stream 280 of the second column including n-propyl alcohol (NPA) and hexanol, and a lower discharge stream 250 of the second column including water. The heat required for the operation of the second column was supplied through a reboiler 230.

An upper end and a lower end of the dividing wall provided in the second column were located at heights of 17% and 83% downwards from the top of the second column, respectively.

In addition, the first side discharge stream and the second side discharge stream were discharged at height of 22% and 45% downwards from the top of the second column, respectively.

At this time, a ratio (yield) of a mass flow rate of the isopropyl alcohol included in the first side discharge stream of the second column to a mass flow rate of the isopropyl alcohol included in the feed 10 was 99.3%, and thus, there was no need for additional distillation of the second side discharge stream of the second column to increase the yield of isopropyl alcohol.

The first side discharge stream including the azeotrope of isopropyl alcohol and water was supplied to an IPA recovery column to finally obtain isopropyl alcohol in the presence of an azeotropic agent.

The reboiler duties of the respective columns in this case were shown in Table 1.

### Comparative Example 1

A process of purifying isopropyl alcohol was performed according to a process diagram of FIG. 2.

A lower discharge stream 150 of a first column having the same composition as that of Example 1 was obtained from the same feed 10 as in Example 1, and then introduced into a second column 200.

The second column 200 of Comparative Example 1 was a column not provided with a dividing wall, and as in Example 1, the stream was separated, by distillation of the second column 200, into each of four discharge streams: an upper discharge stream 260 of the second column including acetone, a first side discharge stream 290 of the second column including an azeotrope of isopropyl alcohol and water, a second side discharge stream 280 of the second column including n-propyl alcohol (NPA) and hexanol, and a lower discharge stream 250 of the second column including water.

The energy required for the operation of the first to third columns was supplied through reboilers provided below the first to third columns.

In this case, as a large amount of isopropyl alcohol was included in the second side discharge stream 280 of the second column and was discharged without effectively separating the heavy by-products and water, it was impossible to achieve a yield of 99% of isopropyl alcohol included in the first side discharge stream 290 of the second column by operating only the second column 200, and accordingly, the third column 300 was required to further purify the isopropyl alcohol in the second side discharge stream 280 of the second column and to separate the heavy by-products and water.

Specifically, the second side discharge stream 280 of the second column was introduced into the third column 300, a stream including isopropyl alcohol was separated through an upper portion of the third column and supplied again to the second column, water was separated through a lower portion of the third column, and heavy by-products (n-propyl alcohol and hexanol) were separated from a side of the third column.

The reboiler duties of the respective columns in this case were shown in Table 1.

**[Table 1]**

| | Comparative Example 1 | | | Example 1 | |
|---|---|---|---|---|---|
| | First column | Second column | Third column | First column | Second column |
| Reboiler duty in each column (kW) | 22.5 | 66.2 | 11.3 | 10.2 | 53.4 |
| Total reboiler duty (kW) | 100 | | | 63.6 | |
| Energy (%) saving | 0 | | | 36.4 | |

In Table 1, the total energy consumption in Example 1 represents the relative energy consumption when the total energy consumption in Comparative Example 1 is set as 100, and the energy consumption in the reboiler in each column in Example 1 represents the value obtained by distributing the relative energy consumption in proportion to the actual energy consumption.

Referring to Table 1, the energy saving of Example 1 was about 36% compared to that of Comparative Example 1. In Example 1, unlike Comparative Example 1 in which three columns were operated, the same amount of isopropyl alcohol was obtained using only two columns, and at the same time, the overall energy consumption was saved.

**[Detailed Description of Main Elements]**

| | | | |
|---|---|---|---|
| 10: | Feed | | |
| 100: | First column | 200: | Second column |
| 300: | Third column | 110: | Condenser |
| 120: | Layer separator | 130, 230: | Reboiler |
| 150: | Lower discharge stream of first column | 160: | Upper discharge stream of first column |
| 250: | Lower discharge stream of second column | | |
| 260: | Upper discharge stream of second column | | |
| 280: | Second side discharge stream of second column | | |
| 290: | First side discharge stream of second column | | |
| 201: | Top region | 202: | Bottom region |
| 203: | Supply region | 204: | Discharge region |

## Claims

1. A method for purifying isopropyl alcohol, the method comprising:
supplying a feed including isopropyl alcohol, water, a first light by-product, a second light by-product, and a heavy by-product to a first column;
separating the first light by-product through an upper discharge stream of the first column;
supplying a lower discharge stream of the first column including the isopropyl alcohol, water, second light by-product, and heavy by-product to a second column, and separating the lower discharge stream of the first column into each of an upper discharge stream of the second column including the second light by-product, a first side discharge stream of the second column including a mixture of the isopropyl alcohol and water, a second side discharge stream of the second column including the heavy by-product, and a lower discharge stream of the second column including the water; and
introducing the mixture of the isopropyl alcohol and water into an isopropyl alcohol recovery process and separating the isopropyl alcohol and water.

2. The method of claim 1, wherein the upper discharge stream of the first column includes the isopropyl alcohol, water, and first and second light by-products.

3. The method of claim 2, wherein the first light by-product is separated by:
supplying the upper discharge stream of the first column to a condenser and liquefying the upper discharge stream of the first column, and
supplying the liquefied upper discharge stream of the first column to a layer separator to subject the upper discharge stream of the first column to liquid-liquid separation, refluxing a water phase stream including the isopropyl alcohol, water, and second light by-product to the first column, and removing an oil phase stream including the first light by-product.

4. The method of claim 1, wherein the first light by-product includes diisopropyl ether (DIPE).

5. The method of claim 1, wherein the second column includes a dividing wall spaced apart from the bottom and provided in a longitudinal direction of the column,
the second column is partitioned into a top region, a bottom region, a supply region, and a discharge region by the dividing wall,
the first and second side discharge streams of the second column are discharged from the discharge region, and
the first side discharge stream is discharged from the discharge region at a higher position than the second side discharge stream.

6. The method of claim 5, wherein an upper end of the dividing wall is located at a height of 3 to 30% downwards from the top of the second column, and
a lower end of the dividing wall is located at a height of 70 to 95% downwards from the top of the second column.

7. The method of claim 5, wherein the first side discharge stream is discharged at a height of 5 to 33% downwards from the top of the second column, and
the second side discharge stream is discharged at a height of 40 to 80% downwards from the top of the second column.

8. The method of claim 1, wherein a branched stream branched from a portion of the lower discharge stream of the second column including the water is recycled to an upper portion of the first column.

9. The method of claim 8, wherein a mass flow rate of the branched stream recycled to the upper portion of the first column is 0.4 to 1.2 with respect to a mass flow rate of the feed supplied to the first column.

10. The method of claim 1, wherein a boiling point of the second light by-product is 50 to 70°C.

11. The method of claim 10, wherein the second light by-product includes acetone.

12. The method of claim 1, wherein the upper discharge stream of the second column includes 50 to 100 wt% of the second light by-product and a residual mixture of isopropyl alcohol and water.

13. The method of claim 1, wherein a boiling point of the heavy by-product is 85 to 99°C.

14. The method of claim 13, wherein the heavy by-product includes n-propyl alcohol (NPA) and hexanol.
